# EUROPÄISCHE PATENTSCHRIFT

(11) **EP 0 556 218 B1**
(45) Veröffentlichungstag und Bekanntmachung des Hinweises auf die Patenterteilung: **27.12.1995**
(21) Anmeldenummer: 91918681.7
(22) Anmeldetag: 04.11.1991
(51) Int. Cl.: C07C 43/11, C07C 41/03, A61K 31/08, A61K 7/50

(54) **LOKALANÄSTHETIKUM**
LOCAL ANAESTHETIC
ANESTHESIQUE LOCAL

(30) Priorität: 09.11.1990 DE 4035682
(43) Veröffentlichungstag der Anmeldung: 25.08.1993
(73) Patentinhaber: Henkel Kommanditgesellschaft auf Aktien, 40191 Düsseldorf (DE)
(72) Erfinder: ANSMANN, Achim, D-4006 Erkrath (DE); BEHLER, Ansgar, D-4250 Bottrop (DE); FRANKE, Rolf, D-2057 Reinbeck (DE); SCHMERSAHL, Peter, D-2000 Barsbüttel (DE)
(86) Internationale Anmeldenummer: EP9102075
(87) Internationale Veröffentlichungsnummer: WO9208684

(56) Entgegenhaltungen:
- EP-A- 0 158 249
- EP-A- 0 186 453
- EP-A- 0 323 798
- EP-A- 0 339 426
- EP-A- 0 361 617
- DE-A- 944 127
- US-A- 2 828 243
- Chemical Abstracts, Band 78, no. 19, 14 Mai 1973, (Columbus, Ohio, US), Dittmann E.C. : "Structure activity relations in homologous alkyl polyglycol ethers ", siehe Seite 8, Zusammenfassung 119086h, & Naunyn-Schmiedeberg's Arch. Pharmacol. 1973, 276( 2), 199- 210
- Naunyn-Schmiedebergs Arch. exp. Path. u. Pharmak., Band. 247, 1964 H.F. Zipf et al.: "Beziehungen zwischen Alkylkettenlänge, Lipophilie, lokalanaesthetischer und endoanaesthetischer Wirksamkeit bei homologen Alkylpolyglykoläthern"

## Beschreibung

Die Erfindung betrifft ein Gemisch homologer Laurylpolyglycolether mit eingeengter Homologenverteilung, dessen Verwendung als lokalanästhetischer Wirkstoff in therapeutischen Zubereitungen zu Behandlung des menschlichen Körpers und als reizmindernde Komponente in kosmetischen Zubereitungen zur Pflege und Reinigung der Haut und der Haare.

Die lokalanästhetische Wirkung homologer Alkylpolyglycolether ist seit langem bekannt (vgl. z.B. K. Soehring , M. Frahm und K. Mletzko: Arch. int. pharmacodyn. 91 112 (1952) oder H.F. Zipf, E. Ch. Dittmann: Naunyn Schmiedebergs Arch. exp. Path. u. Pharmak. 247, 544 bis 557 (1964). Als wirksamste Verbindungen wurden die Anlagerungsprodukte von ca. 9 Mol Ethylenoxid an n-Laurylalkohol erkannt. Diese Addukte stellen Gemische homologer Polyglycolether dar, deren mittlerer Oxethylierungsgrad bei 9 liegt, die aber eine breite Homologenverteilung aufweisen, wobei die Homologen mit 8, 9 und 10 Glycolethergruppen nicht mehr als insgesamt 30 bis 40 Gew.-% des Gemisches ausmachen. Gleichzeitig sind jeweils mehr als 30 Gew.-% niedrigerer und höherer homologer Polyglycolether und freier Laurylalkohol im Gemisch vorhanden. Ein Produkt dieser Art ist unter der Bezeichnung "Polydocanol" oder "Thesit ^{(R)}" als Lokalanästhetikum im Handel.

Die bekannten Lokalanästhetika vom Typ des Polydocanol sind in ihrer Wirkung begrenzt. Sie haben auch gewisse Nebenwirkungen, insbesondere sind in einzelnen Fallen Kontaktallergien aufgetreten, als deren Auslöser man u.a. auch Nebenprodukte des technischen Polydocanol vermutet hat. Es bestand daher ein Interesse an einem chemisch einheitlicher zusammengesetzten Produkt.

Aus EP-A-158 249 waren medizinische Ölbäder bekannt, die als Emulgator einen Fettalkoholpolyglycolether vom Typ des Polydocanol enthalten. Aus US-A-2,828,243 war ein Gemisch homologer Laurylpolyglycolether mit statistischer Homologenverteilung zur Dampf- und Inhalationstherapie bekannt. Ein Gemisch mit besonders hohem Gehalt an Homologen mit 4 bis 8 Glycolethergruppen ist diesen Druckschriften nicht zu entnehmen.

In der Zwischenzeit sind zahlreiche Verfahren bekanntgeworden, um Alkylpolyglycolether mit enger Homologenverteilung herzustellen. Die nach solchen Verfahren erzeugten Ethylenoxidanlagerungsprodukte bestehen zum größten Teil aus Homologen mit x-1, x und x+1 Glycolethergruppen, wenn x die Zahl der angelagerten Mol Ethylenoxid pro Mol Alkanol ist. Die meisten dieser Verfahren beruhen darauf, daß das Ethylenoxid in Gegenwart eines Katalysators angelagert wird, der ein Oxid, Hydroxid oder Salz eines Erdalkalimetalls ist.

Es wurde nun gefunden, daß Gemische homologer Laurylpolyglycolether der Formel I

H₁₂H₂₅O-(CH₂CH₂O)ₓ-H (I)

in der C₁₂H₂₅ eine lineare primäre Alkylgruppe ist und x einen Mittelwert von 5 bis 7 aufweist, und wobei wenigstens 75 Gew.-% des Homologengemisches aus Homologen mit x = 4 bis 8 Glycolethergruppen bestehen, eine gegenüber dem bekannten Polydocanol unerwartet hohe lokalanästhetische Wirkung aufweisen.

Ein besonders wirksames Lokalanästhetikum ist ein Laurylpolyglycolether der Formel I mit einem mittleren Oxethylierungsgrad x von 6,5, der weniger als 15 Gew.-% Homologe mit einem Ethoxylierungsgrad von 0 bis 4 und weniger als 10 Gew.-% Homologe mit einem Ethoxylierungsgrad von 9 und mehr aufweist.

Die Herstellung des erfindungsgemäßen Gemisches homologer Laurylpolyglycolether der Formel I kann nach einem der bekannten Oxethylierungsverfahren erfolgen, die Oxethylate mit einer engen Homologenverteilung (narrow range ethoxylates) ergeben. Solche Verfahren sind z.B. in EP-A-6105, EP-A-18463, EP-A-20 867, EP-A-33 359, EP-A-46 582, EP-A-46 947, EP-A-82 554, EP-A-95 562 oder EP-A-321 053 sowie in der deutschen Patentanmeldung P 40 03 658.8 beschrieben.

Ein besonders bevorzugtes Verfahren zur Herstellung solcher Oxethylate ist in EP-A-339 426 beschrieben. Nach diesem bevorzugten Herstellungsverfahren wird das erfindungsgemäße Gemisch homologer Laurylpolyglycolether in der Weise hergestellt, daß man 5 bis 7 Mol, bevorzugt 6,5 Mol Ethylenoxid an ein Mol n-Dodecanol-1 in Gegenwart von calcinierten Hydrotalciten als Katalysatoren an lagert. Geringe Mengen an Polyethylenglycol und hochmolekularen Oxethylaten lassen sich durch Filtration aus dem flüssigen Oxethylat entfernen.

Das erfindungsgemäße Gemisch homologer Laurylpolyglycolether der Formel I weist eine sehr hohe lokalanästhetische Wirkung auf. Die Grenzkonzentration in Wasser zur Erreichung des lokalanästhetischen Schwellwertes liegt nur halb so hoch wie die des bekannten Polydocanol (n-Dodecanol-1-poly-(9EO)glycolether-Gemisch). Das Wirkspektrum umfaßt sowohl die äußerliche als auch die endogene lokale Anästhesie. Haupteinsatzgebiet sind daher Antipruriginosa, Antiekzematosa und Hämorrhoidalpräparate.

Wegen der geringen Nebenwirkungen ist außer der rein therapeutischen Anwendung vor allem auch die topische kosmetische Anwendung von Bedeutung. Ein weiterer Erfindungsgegenstand ist daher die Verwendung des erfindungsgemäßen Gemisches homologer Laurylpolyglycolether als reizmindernde Komponente in kosmetischen Zubereitungen zur Pflege und Reinigung der Haut und der Haare.

Solche kosmetischen Zubereitungen sind z.B. Sonnenschutzpräparate, Hautcremes, Duschpräparate, Badezusätze, Flüssigseifen, Shampoos, Deodorantien und schweißhemmende Zubereitungen sowie Mund- und Zahnpflegemittel.

Das erfindungsgemäße Gemisch homologer Laurylpolyglycolether der Formel I hat außerdem sehr gute Emulgatoreigenschaften. Es eignet sich daher ganz besonders zur Herstellung kosmetischer Zubereitungen der obengenannten Art die hautverträgliche, rückfettende Ölkomponenten in emulgierfähiger, emulgierter oder solubilisierter Form enthalten. In diesen Fällen entfalten die Laurylpolyglycolether-Gemische gleichzeitig eine emulgierende und eine reizvermindernde Wirkung.

Eine besonders bevorzugte Ausführungsform der vorliegenden Erfindung sind daher medizinische Badepräparate und Körperreinigungsmittel, die physiologisch verträgliche Ölkomponenten und als Emulgatoren wenigstens ein Gemisch homologer Laurylpolyglycolether der Formel I enthalten. Das erfindungsgemäße Gemisch homologer Laurylpolyglycolether kann dabei in einer Menge von 0,05 bis 1 Gewichtsteil pro Gewichtsteile der Öl- oder Fettkomponente enthalten sein.

Als physiologisch verträgliche Öle eignen sich bevorzugt solche Ölkomponenten und Gemische von Ölen mit Fetten und Wachsen, die bei + 20 °C noch flüssig sind. Beispiele für solche Ölkomponenten sind Oleylalkohol, 2-Octyl-dodecanol, 2-Hexyl-decanol, Paraffinöle, Isopropylmyristat, Isopropylpalmitat, Decyloleat, Isooctylstearat, Oleyloleat, Decylerucat, natürliche flüssige Wachsester wie Spermöl und Jojobaöl und synthetische Analoga, vor alle aber Fettsäuretriglyceride auf Basis von C₈-C₂₂-Fettsäuren, z.B. Capryl- und/oder Caprinsäure-Triglycerid, Ölsäuretriglycerid, flüssige natürliche Triglyceridöle, wie z.B. Olivenöl, Sojaöl, Sonneblumenöl, Erdnußöl, Mandelöl, Avocadoöl, Haselnußöl und Weizenkeimöl. Schließlich Dicarbonsäureester wie z.B. Dibutylsebacat, Dioctyladipat, die Vollester von Diolen oder Polyolen, z.B. die Fettsäure C₈-C₁₈-Ester des Ethylenglycols, das 1,2-Propylenglycols, des Butandiols, des Hexandiols des Neopentylglycols, des Trimethylolpropans und des Pentaerythrits und die Komplexester aus Dicarbonsäuren, Diolen (oder Polyolen) und Fettsäuren.

Die medizinischen Badepräparate und Körperreinigungsmittel können z.B. als Emulsionskonzentrate vorliegen, die kein oder nur wenig Wasser enthalten und sich beim Zusatz zum Badewasser spontan emulgieren. Sie können aber auch als Emulsionen oder Solubilisate vorliegen, die bereits Wasser als äußere Phase enthalten.

Zur Herstellung der spontan emulgierbaren Emulsionskonzentrate kann es erforderlich sein, weitere Emulgatoren und ggf. Coemulgatoren einzusetzen, um eine gute Verteilbarkeit der Badepräparate im Wasser und eine stabile Emulsionsbildung zu erreichen. Darüber hinaus können auch weitere pharmazeutische, dermatologische oder kosmetische Wirkstoffe, Duftstoffe, Antioxidantien, Verdickungsmittel, Lösungsvermittler, Komplexbildner und Puffersalze zur pH-Wert-Einstellung enthalten sein.
Die folgenden Beispiele sollen den Erfindungsgegenstand näher erläutern:

### Beispiele

1a) Herstellung von Dodecanol-poly-(6,5)-glycolether (NRE) analog EP 339 426 A2, Beispiel 1
Ein handelsüblicher synthetischer Hydrotalcit wurde 8 h bei 500 °C calciniert. Zur Umsetzung eines handelsüblichen Laurylalkohols mit 6,5 Mol Ethylenoxid wurden 1,860 kg Laurylalkohol in einem Druckreaktor vorgelegt und mit 24 g des zuvor erhaltenen calcinierten Hydrotalcits versetzt. Der Reaktor wurde mit Stickstoff gespült und 30 Min. lang bei einer Temperatur von 100 °C evakuiert. Anschließend wurde die Temperatur auf 180 °C gesteigert und 2,860 kg Ethylenoxid bei einem Druck von 4 bis 5 bar aufgedrückt. Nach Beendigung der Reaktion ließ man 30 Min. nachreagieren. Nach dem Abfiltrieren von suspendiertem Katalysator erhielt man das gewünschte Gemisch homologer Alkylpolyglycolether. Das Produkt hatte folgende Kenndaten:

| | |
|---|---|
| Hydroxylzahl: | 122 |
| n_{D}⁵⁰: | 1,4449 |
| D₇₀: | 0,950 |
| Trübungstemperatur: | 56 °C (1 Gew.-% in Wasser) |
| pH-Wert: | 6,8 (1 Gew.-% in Wasser) |

1b) Herstellung von Dodecanol-poly(6,5)-glycolether analog deutscher Patentanmeldung P 40 03 658.8.
In 235,7 g Dodecanol (1,25 Mol) wurden 6 g Strontiumphosphat, welches bei 260°C calciniert war, aufgelöst. Die Lösung wurde in einen Autoklaven überführt, der mit Stickstoff gespült und dann 30 Minuten bei 100°C evakuiert wurde. Anschließend wurde unter Erwärmung auf maximal 180°C unter einem Druck von 5 bar Ethylenoxid aufgedrückt. Es wurden innerhalb von 4 Stunden portionsweise 364,3 g (8,2 Mol) Ethylenoxid aufgenommen.
Nach Beendigung der Ethylenoxidzugabe wurde die Temperatur noch 30 Minuten bei 170°C gehalten (Nachreaktionszeit). Dann wurde Kühlung auf Raumtemperatur der Katalysator über einen Druckfilter (max. 3 bar) abgetrennt. Die Ausbeute betrug 557 g. Die Hydroxylzahl betrug 120,2. Die Produkte hatten folgende Homologenverteilung

| | 1a | 1b |
|---|---|---|
| Dodecanol | 0,1 Gew.-% | 1,5 Gew.-% |
| Dodecanol-monoglycolether | 0,2 Gew.-% | 1,9 Gew.-% |
| Dodecanol-diglycolether | 1,0 Gew.-% | 2,9 Gew.-% |
| Dodecanol-tetraglycolether | 8,0 Gew.-% | 8,9 Gew.-% |
| Dodecanol-pentaglycolether | 18,0 Gew.-% | 14,8 Gew.-% |
| Dodecanol-hexaglycolether | 25,0 Gew.-% | 19,0 Gew.-% |
| Dodecanol-heptaglycolether | 22,0 Gew.-% | 19,2 Gew.-% |
| Dodecanol-octaglycolether | 13,0 Gew.-% | 15,7 Gew.-% |
| Dodecanol-nonaglycolether | 6,0 Gew.-% | 9,8 Gew.-% |
| Dodecanol-decaglycolether | 3,0 Gew.-% | 3,5 Gew.-% |
| - höhere Polyglycolether | 3,7 Gew.-% | 2,8 Gew.-% |

2. Lokalanästhetische Wirkung
Die Prüfung der lokalanästhetischen Wirkung erfolgte durch Corneareflex-Prüfung am Kaninchenauge. Diese Methode wurde zuerst von M.J. Regnier, (C.R. Acad. Sci. Paris 177, 558 bis 560, 1923) publiziert (vgl. auch Th. Eckert, E. Wachtel, Arzneimittelforschung / Drug Res. 33, 98 bis 100, 1983). Die Methode beruht darauf, daß die Zahl der mit Hilfe einer Reizborste ausgeübten Reize bis zum ersten vollständigen Lidschlag proportional dem Grade der erreichten Anästhesie ist. Erfolgt nach 100 Reizungen (im Abstand von 4 Sekunden) kein Lidschluß, so liegt Vollanästhesie vor.
Es wurde die Grenzkonzentration im Wasser bestimmt, bei welcher ein lokalanästhetischer Schwellwert (50 Reizungen ohne Lidschlag) erreicht wird. Diese Grenzkonzentration lag für das Produkt gemäß Beispiel 1 bei 1:145 000 (ca. 6,9 · 10⁻⁶). Für Polydocanol (Thesit ^{(R)}, n-Dodecanol-1 +9 EO, normale Homologenverteilung) liegt die Grenzkonzentration bei 1:70 000 (1,4 · 10⁻⁵).
3. Anwendungsbeispiel
Es wurde ein spontanemulgierfähiges, erfindungsgemäßes Badeöl folgender Zusammensetzung hergestellt:

| | |
|---|---|
| Sojabohnenöl: | 85 Gew.-% |
| Produkt gemäß Beispiel 1: | 15 Gew.-% |

Zum Vergleich wurde ein Badeöl aus 85 Gew.-% Sojabohnenöl und 15 Gew.-% Polydocanol (normale Homologenverteilung) hergestellt. Beide Produkte wurden in Wasser dispergiert und die lokalanasthetische Wirkung einer Verdünnungsreihe in der unter 2 beschriebenen Weise bis zum Erreichen der Grenzkonzentration bestimmt.

| | |
|---|---|
| Erfindungsgemäßes Badeöl : | Grenzkonzentration 1:22 000 |
| Vergleichsprodukt mit Polydocanol: | Grenzkonzentration 1: 8 000 |

## Patentansprüche

1. Gemisch homologer Laurylpolyglycolether der Formel
C₁₂H₂₅O(CH₂-CH₂O)ₓ-H (I)
in der C₁₂H₂₅ eine lineare, primäre Alkylgruppe ist und x einen Mittelwert von 5 bis 7 aufweist, wobei wenigstens 75 Gew.-% des Homologengemisches aus Homologen mit x = 4 bis 8 Glycolethergruppen bestehen, zur Anwendung als lokalanästhetischer Wirkstoff.

2. Verfahren zur Herstellung eines Gemisches homologer Laurylpolyglycolether der Formel I gemäß Anspruch 1, dadurch gekennzeichnet, daß man 6,5 Mol Ethylenoxid an ein Mol n-Dodecanol-1 in Gegenwart eines calcinierten Hydrotalcits als Katalysator anlagert.

3. Verwendung eines Gemisches homologer Laurylpolyglycolether gemäß Anspruch 1 als lokalanästhetischer Wirkstoff zur Herstellung therapeutischer Zubereitungen zur Behandlung des menschlichen Körpers.

4. Verwendung eines Gemisches homologer Laurylpolyglycolether gemäß Anspruch 1 als reizmindernde Komponente in kosmetischen Zubereitungen zur Pflege und Reinigung der Haut und der Haare.

## Claims

1. A mixture of homologous lauryl polyglycol ethers corresponding to the following formula
H₁₂H₂₅O-(CH₂CH₂O)ₓ-H (I)
in which H₁₂H₂₅ is a linear primary alkyl group and x has an average value of 5 to 7, at least 75% by weight of the homolog mixture consisting of homologs with x = 4 to 8 glycol ether groups, for use as a local anaesthetic agent.

2. A process for the production of the mixture of homologous lauryl polyglycol ethers of formula I claimed in claim 1, characterized in that 6.5 mol ethylene oxide are added onto 1 mol n-dodecan-1-ol in the presence of a calcined hydrotalcite as catalyst.

3. The use of the mixture of homologous lauryl polyglycol ethers claimed in claim 1 as a local anaesthetic agent for the production of therapeutic preparations for treatment of the human body.

4. The use of the mixture of homologous lauryl polyglycol ethers claimed in claim 1 as an irritation-reducing component in cosmetic hair and skin care and cleaning preparations.

## Revendications

1. Mélange de laurylpolyglycoléthers homologues de formule
C₁₂H₂₅O(CH₂-CH₂O)ₓ-H (I)
dans laquelle C₁₂H₂₅ représente un groupe alkyle primaire linéaire et X a une valeur moyenne de 5 à 7, le mélange d'homologues étant constitué à raison d'au moins 75% par des homologues dans lesquels X correspond à 4 à 8 groupes glycoléthers, à utiliser comme agent anesthésique local.

2. Procédé de préparation d'un mélange de laurylpolyglycoléthers homologues de formule I selon la revendication 1, caractérisé en ce qu'on fixe 6,5 moles d'oxyde d'éthylène sur une mole de n-dodécanol-1 en présence d'hydrotalcite calcinée en tant que catalyseur.

3. Utilisation d'un mélange de laurylpolyglycoléthers homologues selon la revendication 1 en tant qu'agent anesthésique local pour l'obtention de préparations thérapeutiques en vue du traitement du corps humain.

4. Utilisation d'un mélange de laurylpolyglycoléthers homologues selon la revendication 1 en tant que constituant diminuant l'irritation dans des préparations cosmétiques pour le soin de la peau et de la chevelure.
